(19) Europäisches Patentamt
European Patent Office
Office européen des brevets

(11) **EP 4 354 362 A1**

(12) **EUROPEAN PATENT APPLICATION**
published in accordance with Art. 153(4) EPC

(43) Date of publication:
**17.04.2024 Bulletin 2024/16**

(21) Application number: **22820208.1**

(22) Date of filing: **07.06.2022**

(51) International Patent Classification (IPC):
**G06N 99/00** (2019.01)

(52) Cooperative Patent Classification (CPC):
**G06N 99/00**

(86) International application number:
**PCT/JP2022/022916**

(87) International publication number:
**WO 2022/260029 (15.12.2022 Gazette 2022/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.06.2021 JP 2021097381**

(71) Applicant: **Resonac Corporation**
**Tokyo (JP)**

(72) Inventors:
• **SAKAGUCHI, Suguru**
**Tokyo 105-8518 (JP)**
• **OKUNO, Yoshishige**
**Tokyo 105-8518 (JP)**

(74) Representative: **Strehl Schübel-Hopf & Partner**
**Maximilianstrasse 54**
**80538 München (DE)**

(54) **INFORMATION PROCESSING SYSTEM, MATERIAL COMPOSITION SEARCHING METHOD, MATERIAL COMPOSITION SEARCHING DEVICE, AND PROGRAM**

(57) An information processing system having an annealing computing device configured to use an Ising model, and a material composition searching device configured to convert a combinatorial optimization problem of a material composition asymptotically approaching a target physical property value into an Ising model and cause the computing device to solve the combinatorial optimization problem, includes an input reception unit configured to receive an input of a target value of at least one physical property, a conversion unit configured to convert a mathematical expression that formulates a combinatorial optimization problem of a material composition that asymptotically approaches the target value from a mixed material of materials having known physical property values, into the Ising model in a data format usable by the computing device, an optimum solution computing unit configured to compute an optimum solution of the material composition asymptotically approaching the target value, using the Ising model, and an output control unit configured to output the computed optical solution of the material composition asymptotically approaching the target value, wherein the mathematical expression formulates the material composition that asymptotically approaches the target value, so that the optimum solution is less likely computed as a number of materials included in the material composition increases, and is more likely computed as the number of materials included in the material composition decreases.

FIG.5

```
START
   │
   ▼                                          S10
┌──────────────────────────────────────────────┐
│ RECEIVE INPUTS OF OBJECTIVE PHYSICAL           │
│ PROPERTY OF MIXED SOLVENT TO BE OPTIMIZED,     │
│ TARGET VALUE OF OBJECTIVE PHYSICAL PROPERTY,   │
│ AND WEIGHT CONSTANTS                           │
│ (FOR EXAMPLE, VISCOSITY, HSP, COST, ETC.)      │
└──────────────────────────────────────────────┘
   │                                          S12
   ▼
┌──────────────────────────────────────────────┐
│ RECEIVE INPUT OF SOLVENT INFORMATION           │
│ OF SOLVENTS TO BE MIXED                         │
└──────────────────────────────────────────────┘
   │                                          S14
   ▼
┌──────────────────────────────────────────────┐
│ CONVERT INTO ISING MODEL                       │
│ (COMPUTE MATRIX ELEMENTS OF FORMULA OF         │
│  ISING MODEL) HAVING DATA FORMAT USABLE IN     │
│ ANNEALING COMPUTER (SUITABLE FOR ANNEALING     │
│ COMPUTER), USING OBJECTIVE PHYSICAL PROPERTY,  │
│ TARGET VALUE OF OBJECTIVE PHYSICAL PROPERTY,   │
│ AND FORMULA (1)                                │
└──────────────────────────────────────────────┘
   │                                          S16
   ▼
┌──────────────────────────────────────────────┐
│ COMPUTE OPTIMUM SOLUTION OF SOLVENT            │
│ COMPOSITION, USING ISING MODEL HAVING DATA     │
│ FORMAT USABLE IN ANNEALING COMPUTER            │
└──────────────────────────────────────────────┘
   │                                          S18
   ▼
┌──────────────────────────────────────────────┐
│ OUTPUT MIXED SOLVENT OF OPTIMUM SOLUTION,      │
│ AND PHYSICAL PROPERTY VALUE OF                 │
│ OBJECTIVE PHYSICAL PROPERTY OF MIXED SOLVENT   │
└──────────────────────────────────────────────┘
   │
   ▼
  END
```

EP 4 354 362 A1

**Description**

TECHNICAL FIELD

The present disclosure relates to information processing systems, material composition searching methods, material composition searching devices, and programs.

BACKGROUND ART

For example, there is a combinatorial optimization problem for selecting an optimum combination from combinations of various elements, such as a searching for a material composition having optimum physical properties. It may not be possible to solve the combinatorial optimization problem within a practical length of time, because the number of combinations increases explosively as the number of elements increases. In a case where 100 kinds of materials are combined in increments of 1% to generate a mixed material, for example, the number of combinations becomes $5 \times 10^{58}$.

An annealing machine using the Ising model has been proposed as an architecture specialized in solving such a combinatorial optimization problem. The annealing machine can efficiently solve the combinatorial optimization problem converted into the Ising model.

Conventionally, there is known a technique for optimizing the thermophysical properties of a mixed refrigerant by using a computer architecture specialized in the combinatorial optimization problem (refer to Non-Patent Document 1, for example).

PRIOR ART DOCUMENTS

NON-PATENT DOCUMENTS

Non-Patent Document 1: "Optimization of Thermophysical Properties for Mixed Refrigerants with Digital Annealer", No. 19-303, The Japan Society of Mechanical Engineers, The Proceedings of the Thermal Engineering Conference 2019 [2019.10.12-13, Nagoya]

DISCLOSURE OF THE INVENTION

PROBLEM TO BE SOLVED BY THE INVENTION

For example, in a case where a combinatorial optimization problem of a material composition asymptotic to (approximating) a target physical property value is solved by the annealing machine, a composition of a mixed material (a material included in the mixed material, and a ratio of this material) asymptotic to the target physical property value is obtained as a result. However, even if the composition of the mixed material obtained as the result is asymptotic to the target physical property value, there are cases where it is difficult to handle the result in practice, such as when the number of materials included in the mixed material is large.

One object of the present disclosure is to provide an information processing system, a material composition searching method, a material composition searching device, and a program capable of varying an optimum solution of a combinatorial optimization problem of a material composition asymptotic to a target physical property value, according to a number of materials included in the material composition.

MEANS OF SOLVING THE PROBLEM

The present disclosure includes the following configurations.

[1] An information processing system having an annealing computing device configured to use an Ising model, and a material composition searching device configured to convert a combinatorial optimization problem of a material composition asymptotically approaching a target physical property value into an Ising model and cause the computing device to solve the combinatorial optimization problem, characterized in that there are provided:

an input reception unit configured to receive an input of a target value of at least one physical property;
a conversion unit configured to convert a mathematical expression that formulates a combinatorial optimization problem of a material composition that asymptotically approaches the target value from a mixed material of materials having known physical property values, into the Ising model in a data format usable by the computing device;

an optimum solution computing unit configured to compute an optimum solution of the material composition asymptotically approaching the target value, using the Ising model; and

an output control unit configured to output the computed optical solution of the material composition asymptotically approaching the target value,

wherein the mathematical expression formulates the material composition that asymptotically approaches the target value, so that the optimum solution is less likely computed as a number of materials included in the material composition increases, and is more likely computed as the number of materials included in the material composition decreases.

[2] The information processing system according to [1], characterized in that the mathematical expression formulates the combinatorial optimization problem of the material composition asymptotically approaching the target value using an energy function including

a cost function that outputs a value so that the value is more likely computed as the optimum solution as the physical property value of the mixed material becomes closer to the target value, and the value is less likely computed as the optimum solution as the physical property value of the mixed material becomes farther from the target value,

a first constraint condition that outputs a value so that the value is not computed as the optimum solution in a case where a total of mixing ratios of materials included in the mixed material does not become 100%, and

a second constraint condition that outputs a value so that the value is more likely computed as the optimum solution as the number of materials included in the material composition decreases, and the value is less likely computed as the optimum solution as the number of materials included in the material composition increases.

[3] The information processing system according to [1] or [2], characterized in that the mathematical expression is the following formula (1),
[Math. 1]

$$E = \sum_{k=1}^{L} \alpha_k \cdot \left\{ \left( \sum_{i=1}^{N} D_{k,i} r_i \right) - D_{k,0} \right\}^2 + \beta \left\{ \left( \sum_{i=1}^{N} r_i \right) - 1 \right\}^2 + \gamma \left\{ N - \sum_{l=1}^{N} \left( \prod_{j}^{m} n_{i,j} \right) \right\} \cdots (1)$$

where in the formula (1),

L denotes a number of objective physical properties to be optimized,
N denotes a number of solvents with known physical property values,
$D_{k,i}$ denotes a physical property value of a k-th objective physical property of a solvent i to be optimized,
$r_i$ denotes the following formula (2) in which the mixing ratio (component ratio) of the solvent i is expressed in binary,
[Math. 2]

$$r_i = -\sum_{j=1}^{m} c_j (n_{i,j} - 1) \cdots (2)$$

$D_{k,0}$ denotes a target value of the k-th objective physical property of the mixed solvent to be optimized,
$n_{i,j}$ denotes a number "0" or "1" for a case where the mixing ratio of the solvent i is expressed in binary,
$c_j$ denotes a coefficient for the case where the mixing ratio of the solvent i is expressed in binary, and
$\alpha_k$, $\beta$, and $\gamma$ denote weight constants.

[4] The information processing system according to [3], characterized in that the optimum solution computing unit computes a combination of $n_{i,j}$ with which the energy function of the formula (1) becomes a minimum value, as the optimum solution of the material composition asymptotically approaching the target value, using the Ising model obtained by converting the formula (1) into the data format usable by the computing device.
[5] The information processing system according to any one of [1] to [4], characterized in that

the input reception unit receives, from a user, a selection of a target value of at least one physical property, and a selection of a material to be used as the material composition of the mixed material from a plurality of materials having the at least one physical property with a known physical property value, and

the output control unit converts the optimum solution of the material composition computed by the optimum solution computing unit into at least one of a material included in the material composition, a mixture ratio of the material, and a physical property value of the mixed material.

[6] A material composition searching method executed by an information processing system having an annealing computing device configured to use an Ising model, and a material composition searching device configured to convert a combinatorial optimization problem of a material composition asymptotically approaching a target physical property value into an Ising model and cause the computing device to solve the combinatorial optimization problem, characterized in that there are provided:

step of receiving an input of a target value of at least one physical property;
step of converting a mathematical expression that formulates a combinatorial optimization problem of a material composition that asymptotically approaches the target value from a mixed material of materials having known physical property values, into the Ising model in a data format usable by the computing device;
step of computing an optimum solution of the material composition asymptotically approaching the target value, using the Ising model; and
step of outputting the computed optical solution of the material composition asymptotically approaching the target value,
wherein the mathematical expression formulates the material composition that asymptotically approaches the target value, so that the optimum solution is less likely computed as a number of materials included in the material composition increases, and is more likely computed as the number of materials included in the material composition decreases.

[7] A material composition searching device that is coupled to an annealing computing device configured to use an Ising model via a communication network, and converts a combinatorial optimization problem of a material composition asymptotically approaching a target physical property value into an Ising model and causes the computing device to solve the combinatorial optimization problem, characterized in that there are provided:

an input reception unit configured to receive an input of a target value of at least one physical property;
a conversion unit configured to convert a mathematical expression that formulates a combinatorial optimization problem of a material composition that asymptotically approaches the target value from a mixed material of materials having known physical property values, into the Ising model in a data format usable by the computing device;
a coordination unit configured to transmit the converted Ising model to the computing device, and receive, from the computing device, an optimum solution of a material composition asymptotically approaching the target value computed by the computing device; and
an output control unit configured to output the received optical solution of the material composition asymptotically approaching the target value,
wherein the mathematical expression formulates the material composition that asymptotically approaches the target value, so that the optimum solution is less likely computed as a number of materials included in the material composition increases, and is more likely computed as the number of materials included in the material composition decreases.

[8] A program for causing a material composition searching device that is coupled to an annealing computing device configured to use an Ising model via a communication network, and converts a combinatorial optimization problem of a material composition asymptotically approaching a target physical property value into an Ising model and causes the computing device to solve the combinatorial optimization problem, to function as:

an input reception unit configured to receive an input of a target value of at least one physical property;
a conversion unit configured to convert a mathematical expression that formulates a combinatorial optimization problem of a material composition that asymptotically approaches the target value from a mixed material of materials having known physical property values, into the Ising model in a data format usable by the computing device;
a coordination unit configured to transmit the converted Ising model to the computing device, and receive, from the computing device, an optimum solution of a material composition asymptotically approaching the target value computed by the computing device; and

an output control unit configured to output the received optical solution of the material composition asymptotically approaching the target value,

wherein the mathematical expression formulates the material composition that asymptotically approaches the target value, so that the optimum solution is less likely computed as a number of materials included in the material composition increases, and is more likely computed as the number of materials included in the material composition decreases.

EFFECTS OF THE INVENTION

According to the present disclosure, it is possible to vary an optimum solution of a combinatorial optimization problem of a material composition asymptotic to a target physical property value, according to a number of materials included in the material composition.

BRIEF DESCRIPTION OF DRAWINGS

[FIG. 1] FIG. 1 is a diagram illustrating a configuration of an example of an information processing system according to one embodiment.

[FIG. 2] FIG. 2 is a diagram illustrating a hardware configuration of an example of a computer according to one embodiment.

[FIG. 3] FIG. 3 is a diagram illustrating a structure of an example of solvent information with known physical property values.

[FIG. 4] FIG. 4 is a diagram illustrating a configuration of an example of the information processing system according to one embodiment.

[FIG. 5] FIG. 5 is a flow chart illustrating an example of a processing procedure of a material composition searching method of the information processing system according to one embodiment.

[FIG. 6] FIG. 6 is a diagram illustrating a structure of examples of combinations of solvents that become an optimum solution, and mixing ratios.

[FIG. 7] FIG. 7 is a diagram illustrating an image of an example of a material composition search result screen.

MODE OF CARRYING OUT THE INVENTION

Next, embodiments of the present invention will be described in detail. The present invention is not limited to the following embodiments. In one embodiment, an optimization of a mixed solvent including solvents having known physical property values will be described, as an example of an optimization of a mixed material including materials having known physical property values.

<System Configuration>

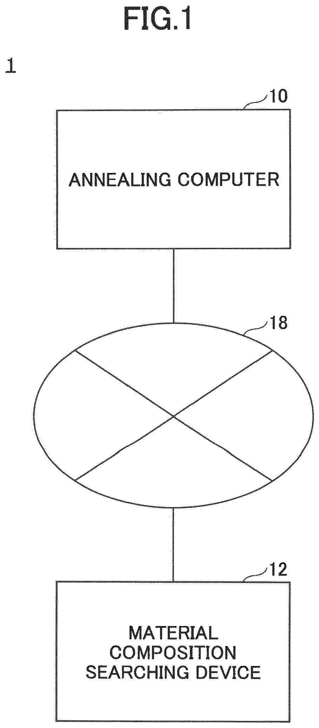

FIG. 1 is a diagram illustrating a configuration of an example of an information processing system according to one embodiment. An information processing system 1 of FIG. 1 includes an annealing computer 10, and a material composition searching device 12. The annealing computer 10 and the material composition searching device 12 are connected via a communication network 18, such as a local area network (LAN), the Internet, or the like, and are communicable with each other to perform data communication.

The annealing computer 10 is an annealing machine using an Ising model, and is an example of an annealing computing device using the Ising model. The annealing computer 10 may be implemented in a quantum computer, or may be implemented in a digital annealer (registered trademark) having a computer architecture in which the annealing is implemented in a digital circuit.

The annealing machine solves the combinatorial optimization problem that is reduced to the Ising model by a convergence operation of the Ising model. The Ising model is a statistical mechanical model representing a behavior of a magnetic body, and has properties such that a spin state is updated by an interaction between spins of the magnetic body so as to minimize an energy (Hamiltonian), and the energy is finally minimized. The annealing machine reduces the combinatorial optimization problem to the Ising model, and obtains the state where the energy is minimized, to thereby obtain the state as an optimum solution of the combinatorial optimization problem.

The material composition searching device 12 is an information processing device that can be operated by a user, such as a PC or the like. The material composition searching device 12 may be an information processing terminal that can be operated by the user, such as a tablet terminal, a smartphone, or the like. The material composition searching device 12 receives an input of information that is required for causing the annealing machine to solve the combinatorial optimization problem reduced to the Ising model, and causes the annealing computer 10 to solve the Ising model.

The material composition searching device 12 receives the optimum solution of the combinatorial optimization problem solved by the annealing computer 10, and outputs the optimum solution so that the optimum solution can be checked by the user, by displaying the optimum solution on a display device, for example.

The information processing system 1 of FIG. 1 is an example, and may further include a user terminal (not illustrated) operable by the user, and the user may make access to and use the material composition searching device 12 from the user terminal, for example.

In addition, the annealing computer 10 may be implemented in a cloud computing service. For example, the annealing computer 10 may be usable by calling an application programming interface (API) via the communication network 18. Further, the annealing computer 10 is not limited to a computer implemented in the cloud computing service, and may be a computer implemented in an on-premises computer, or in an off-premises computer operated by another company. The annealing computer 10 may be implemented in a plurality of computers. The information processing system 1 in FIG. 1 may have a configuration selected from various system configuration examples according to applications and purposes thereof.

<Hardware Configuration>

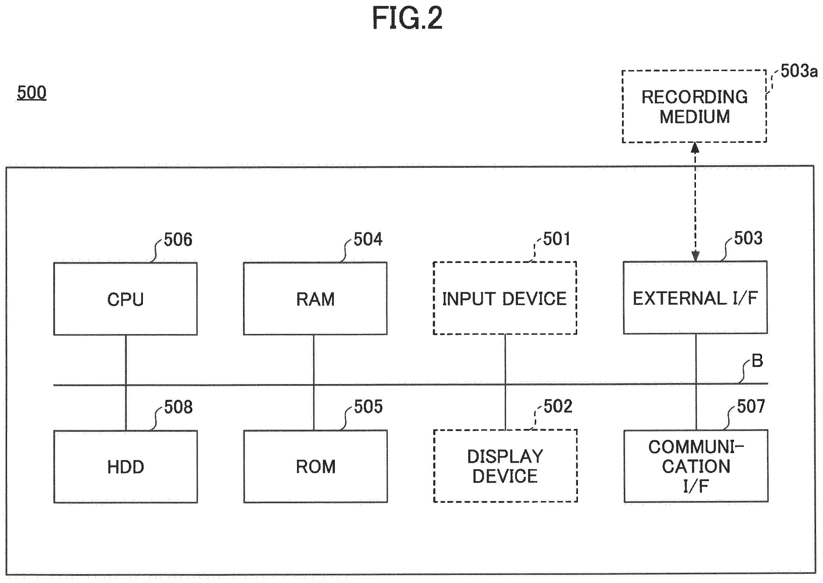

The material composition searching device 12 illustrated in FIG. 1 is implemented in a computer 500 having a hardware configuration illustrated in FIG. 2, for example.

FIG. 2 is diagram illustrating the hardware configuration of an example of the computer according to the one embodiment. The computer 500 of FIG. 2 includes an input device 501, a display device 502, an external I/F 503, a RAM 504, a ROM 505, a CPU 506, a communication I/F 507, a HDD 508, or the like, which are connected to each other via a bus B. The input device 501 and the display device 502 may be connected and used when required.

The input device 501 is a touchscreen panel, operation keys or buttons, a keyboard, a mouse, or the like used by the user to input various signals. The display device 502 includes a display, such as a liquid crystal display, an organic EL display, or the like for displaying a screen, a speaker for outputting sound data, such as voice, sound, or the like, and the like. The communication I/F 507 is an interface used by the computer 500 to perform the data communication.

In addition, the HDD 508 is an example of a non-volatile storage device that stores programs and data. The stored programs and data include an operating system (OS) that is basic software for controlling the entire computer 500, applications for providing various functions on the OS, or the like. The computer 500 may use a drive device (for example, a solid state drive (SSD) or the like) using a flash memory as a storage media, in place of the HDD 508.

The external I/F 503 is an interface provided with respect to an external device. Examples of the external device include a recording medium 503a or the like. Thus, the computer 500 can read from and/or write to the recording medium 503a via the external I/F 503. Examples of the recording medium 503a include a flexible disk, a CD, a DVD, an SD memory card, a USB memory, or the like.

The ROM 505 is an example of a non-volatile semiconductor memory (storage device) that can retain programs and data even when the power is turned off. The ROM 505 stores programs and data, such as a BIOS that is executed when the computer 500 is started, OS settings, network settings, or the like. The RAM 504 is an example of a volatile semiconductor memory (storage device) that temporarily stores the programs and data.

The CPU 506 is an arithmetic unit that reads the programs and data from a storage device, such as the ROM 505, the HDD 508, or the like into the RAM 504, and executes a process to control the entire computer 500 or implement functions thereof. The material composition searching device 12 according to one embodiment can implement various functions that will be described later. A description of a hardware configuration of the annealing computer 10 will be omitted.

<Example of Problem to be Solved as Combinatorial Optimization Problem>

Hereinafter, an example will be described in which a plurality of solvents having known physical property values are mixed, and a mixing ratio (component ratio) of a mixed solvent asymptotic to (approximating) target values of objective physical properties, is solved as a combinatorial optimization problem.

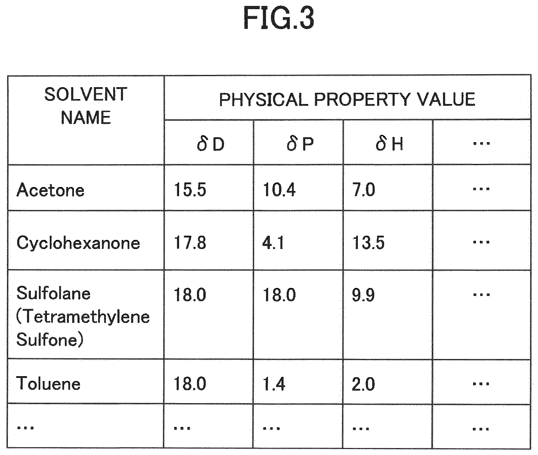

For example, in this embodiment, the plurality of solvents having the known physical property values illustrated in FIG. 3 are used. FIG. 3 is a diagram illustrating a structure of an example of solvent information with known physical property values. In the solvent information of FIG. 3, the property values of one or more physical properties are recorded for each solvent identified by a solvent name. In FIG. 3, the physical values of three physical properties $\delta D$, $\delta P$, and $\delta H$ are recorded as examples.

In this embodiment, it is assumed that the objective physical property is selected from the solvent information illustrated in FIG. 3. It is also assumed that the physical values of the objective physical properties of the mixed solvent are computed according to the following formula (3).

$$\text{Physical property value of mixed solvent} =$$
$$\Sigma(\text{Physical property value of single solvent} \times \text{Mixing}$$
$$\text{ratio}) \quad --- \quad (3)$$

In this embodiment, a problem to be solved as a combinatorial optimization problem is to mix a plurality of kinds of solvents among the solvents included in the solvent information of FIG. 3 in increments of 1%, for example, and to search for a mixing ratio of a mixed solvent asymptotic to target values of objective physical properties.

In this embodiment, the problem to be solved is formulated according to the following formula (1).

[Math. 3]

$$E = \sum_{k=1}^{L} \alpha_k \cdot \left\{ \left( \sum_{i=1}^{N} D_{k,i} r_i \right) - D_{k,0} \right\}^2 + \beta \left\{ \left( \sum_{i=1}^{N} r_i \right) - 1 \right\}^2 + \gamma \left\{ N - \sum_{i=1}^{N} \left( \prod_{j}^{m} n_{i,j} \right) \right\} \cdots (1)$$

In the formula (1),

E denotes an energy function (evaluation function), L denotes the number of physical properties (objective physical properties) to be optimized,
N denotes the number of solvents with known physical property values,
$D_{k,i}$ denotes the physical property value of a k-th objective physical property of a solvent i to be optimized,
$r_i$ denotes the following formula (2) in which a mixing ratio (component ratio) of the solvent i is expressed in binary,
[Math. 4]

$$r_i = - \sum_{j=1}^{m} c_j (n_{i,j} - 1) \cdots (2)$$

$D_{k,0}$ denotes a target value of the k-th objective physical property of the mixed solvent to be optimized,
$n_{i,j}$ denotes a number "0" or "1" for a case where the mixing ratio of the solvent i is expressed in binary, and is "1" when the solvent i is included in a ratio $c_j$, and is "0" when the solvent i is not included in the ratio $c_j$,
$c_j$ denotes a coefficient for the case where the mixing ratio of the solvent i is expressed in binary, and
$\alpha_k$, $\beta$, and $\gamma$ denote weight constants.

The first term on the right side of the formula (1) described above decreases when the physical property values of the objective physical properties of the plurality of mixed solvents computed according to the formula (3) described above approach the target values of the respective objective physical properties. The second term on the right side of the formula (1) described above is a term that becomes "0" (becomes small) when the total value of the mixing ratios (percentages) of the solvents included in the mixed solvent is 1 (100%), and is the constraint term.

In the example of the formula (2) described above, a number of nodes, m, of the ratio $c_j$ required to express the mixing ratio of the solvent i, differs depending on the method of expressing the mixing ratio of the solvent i. For example, in a first method of expressing the mixture ratio of the solvent i, the ratio $c_j$ can be expressed by the number of nodes, "m = 50", such as ($c_1$ = 2/100, $c_2$ = 2/100, ..., $c_{50}$ = 2/100). In a second method of expressing the mixture ratio, the ratio $c_j$ can be expressed by the number of nodes, "m = 100", such as ($c_1$ = 1/100, $c_2$ = 1/100, ..., $c_{100}$ = 1/100). In a third method of expressing the mixture ratio, the ratio $c_j$ can be expressed by the number of nodes, "m = 7", such as ($c_1$ = 64/100, $c_2$ = 32/100, $c_3$ = 16/100, $c_4$ = 8/100, $c_5$ = 4/100, $c_2$ = 2/100, $c_7$ = 1/100). In addition, in a fourth method of expressing the mixture ratio, the ratio $c_j$ can be expressed by the number of nodes, "m = 10", such as ($c_2$ = 512/1000, $c_2$ = 256/1000, $c_3$ = 128/1000, $c_4$ = 64/1000, $c_5$ = 32/1000, $c_6$ = 16/1000, $c_7$ = 8/1000, $c_8$ = 4/1000, $c_9$ = 2/1000, and $c_{10}$ = 1/1000) . For example, the third method of expressing the mixing ratio can be used when the mixing is performed in increments of 1%, and 7 bits are required to express the mixing ratio of one solvent. When the mixing is performed in increments of 0.1%, 10 bits are required to express the mixing ratio of one solvent, as illustrated by the fourth method of expressing the mixing ratio.

The third term on the right side of the formula (1) described above is a constraint term that increases as the number (kinds) of solvents included in the mixed solvent increases, and decreases as the number of solvents included in the mixed solvent decreases. For example, the following formula (4) included in the third term on the right side of the formula (1) described above is a formula for computing "0" or "1". In a case where "1" is computed from the formula (4), all $n_{i,j}$ of a certain solvent i are "1", that is, the solvent i is not included. On the other hand, in a case where "0" is computed from the formula (4), one of $n_{i,j}$ of the certain solvent i is "0", that is, the solvent i is included. Thus, "1" is computed from the formula (4) when the solvent i is not included. Because the formula (1) is an m-th order formula with respect to n, the $n_{i,j}$ of the m-th order needs to be reduced to the second order. For example, a seventh order $n_{i,j}$ (7 variables) can be represented by a second order $n_{i,j}$ (12 variables), using an existing technique.

[Math. 5]

$$\prod_{j}^{m} n_{i,j} \quad \cdots(4)$$

Accordingly, the following formula (5), included in the third term on the right side of the formula (1) described above, represents the number of solvents included in the mixed solvent.

[Math. 6]

$$N - \sum_{i=1}^{N} \left( \prod_{j}^{m} n_{i,j} \right) \quad \cdots(5)$$

For example, in the formula (1) described above, it is possible to increase the influence of the number of solvents included in the mixed solvent on the computation of the optimum solution, by increasing the weight constant $\gamma$. In addition, in the formula (1) described above, it is possible to reduce the influence of the number of solvents included in the mixed solvent on the computation of the optimum solution, by decreasing the weight constant $\gamma$.

By decreasing the weight constant $\gamma$ in the formula (1) described above, for example, the formula (1) assumes a minimum value in a case where the objective physical property of the solvent composition is close to the target value, even when the number of solvents included in the mixed solvent increases, and the solvent composition is computed as the optimum solution. In addition, by increasing the weight constant $\gamma$ in the formula (1) described above, for example, the formula (1) assumes the minimum value in a case where the number of solvents included in the mixed solvent is small, even when the objective physical property is slightly deviated from the target value, and the solvent composition is computed as the optimum solution.

Accordingly, the user can vary the solvent composition that is computed as the optimum solution, according to the number of solvents included in the mixed solvent, by adjusting the weight constant $\gamma$ in the formula (1) described above.

The formula (1) that is formulated, is converted into an Ising model in a data format usable by the annealing computer 10, and is thereafter transmitted from the material composition searching device 12 to the annealing computer 10. The annealing computer 10 computes the optimum solution of the solvent composition, using the Ising model in the usable data format.

The computed optimum solution of the solvent composition is transmitted from the annealing computer 10 to the material composition searching device 12. The material composition searching device 12 outputs the optimum solution of the solvent composition received from the annealing computer 10, so that the optimum solution can be checked by the user.

<Configuration>

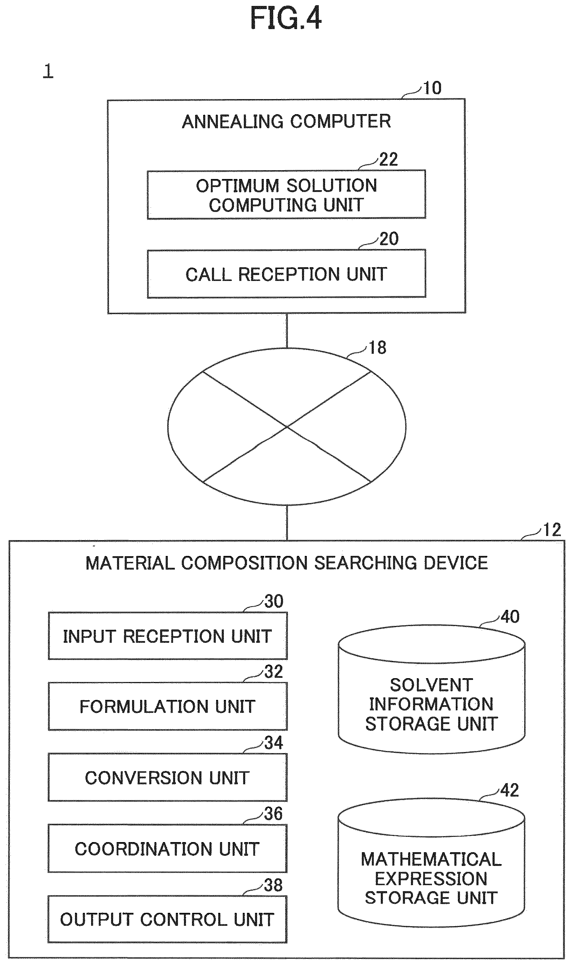

A configuration of the information processing system 1 according to one embodiment will be described. FIG. 4 is a diagram illustrating the configuration of an example of the information processing system according to one embodiment. In the diagram of FIG. 4 illustrating the configuration, portions unnecessary for describing this embodiment are omitted, as appropriate.

The annealing computer 10 of the information processing system 1 illustrated in FIG. 4 includes a call reception unit 20, and an optimum solution computing unit 22. In addition, the material composition searching device 12 includes an input reception unit 30, a formulation unit 32, a conversion unit 34, a coordination unit 36, an output control unit 38, a solvent information storage unit 40, and a mathematical expression storage unit 42.

The input reception unit 30 is an input interface that receives inputs of an objective physical property, and a target value of the objective physical property, from the user. The input reception unit 30 may receive an input of solvent information to be used, from the user. The input reception unit 30 stores the input solvent information in the solvent information storage unit 40.

The formulation unit 32 receives an input of the formula (1), which is a formulation of the problem of mixing the plurality of kinds of solvents among the solvents included in the solvent information of FIG. 3 in increments of 1%, for example, and searching for the mixing ratio of the mixed solvent asymptotic to the target values of the objective physical properties. In addition, the formulation unit 32 receives inputs of the weight constants $\alpha_k$, $\beta$, and $\gamma$ from the user.

The formulation unit 32 receives an input of the formula (1) by a program that defines the formula (1), for example. The formulation unit 32 stores the formula (1) that is input in the mathematical expression storage unit 42.

The conversion unit 34 converts the objective physical property, the target value of the objective physical property, and the formula (1) into the Ising model in the data format that is usable in the annealing computer 10. The coordination unit 36 transmits the Ising model converted by the conversion unit 34 to the annealing computer 10. Further, the coordination unit 36 receives the optimum solution computed by the annealing computer 10.

The output control unit 38 displays the optimum solution received by the coordination unit 36 on the display device 502, so that the optimum solution can be checked by the user. The optimum solution displayed on the display device 502 is displayed as a mixing ratio of the mixed solvent, for example, which is easy for the user to understand. The output control unit 38 may compute the physical property value of the objective physical property of the optimum solution received by the coordination unit 36, and display the computed physical property value of the objective physical property on the display device 502, so that the physical property value can be checked by the user.

The call reception unit 20 receives a call from the material composition searching device 12, and receives the Ising model converted into the data format usable by the material composition searching device 12. The optimum solution computing unit 22 obtains a state where the energy (Hamiltonian) of the Ising model received by the call reception unit 20 is minimized, to thereby search for an optimum solution of a mixing ratio of a mixed solvent having an objective physical property asymptotically approaching a target value. The call reception unit 20 transmits the searched optimum solution to the material composition searching device 12.

The configuration illustrated in FIG. 4 is an example. Various configurations of the information processing system 1 according to this embodiment are conceivable.

<Processes>

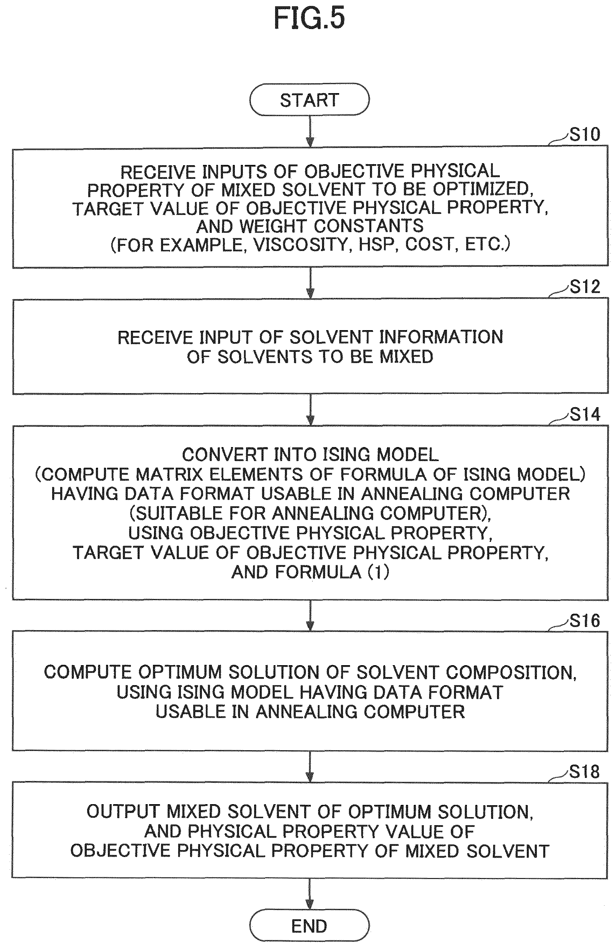

FIG. 5 is a flow chart illustrating an example of a processing procedure of a material composition searching method of the information processing system according to this embodiment. In step S10, the input reception unit 30 of the material composition searching device 12 receives inputs of the objective physical property of the mixed solvent to be optimized, and the target value of the objective physical property, from the user. In this example, it is assumed that inputs of objective physical properties $\delta D$, $\delta P$, and $\delta H$, and target values $(\delta D_0, \delta P_0, \delta H_0) = (18.0, 12.3, 7.2)$ of the objective physical properties $\delta D$, $\delta P$, and $\delta H$ are received from the user. In addition, the input reception unit 30 of the material composition searching device 12 receives inputs of the weight constants $\alpha_k$, $\beta$, and $\gamma$ from the user.

In step S12, the input reception unit 30 of the material composition searching device 12 receives an input of the solvent information on the solvents to be mixed, illustrated in FIG. 3, for example. The solvent information illustrated in FIG. 3 is table data storing physical property values of a plurality of kinds of physical properties of a single solvent to be mixed. The solvent information may be input by selecting a table data file stored in the solvent information storage unit 40. Further, the input reception unit 30 of the material composition searching device 12 may receive a selection of the single solvent to be mixed and an objective physical property of the mixed solvent, from the selected table data file.

In step S14, the formulation unit 32 and the conversion unit 34 of the material composition searching device 12 obtain the Ising model having the data format usable in the annealing computer 10, using the formula (1) in which the problem of mixing a plurality of kinds of solvents in increments of 1%, for example, to search for a mixture ratio of mixed solvents having the objective physical property asymptotically approaching the target value, is formulated, and the objective physical property of the mixed solvent to be optimized and the target value of the objective physical property received as the inputs in step S10.

A technique (library) for converting the formulated formula (1) into a format of an evaluation function of a quadratic unconstrained binary optimization (QUBO), or converting the formulated formula (1) into the Ising model having the data format usable by the annealing computer 10, is an existing technique provided as a Web API or the like. The conversion unit 34 expands the formula (1) described above, to compute a matrix element $Q_{i,j}$ of the Ising model illustrated in the following formula (6), and transmits the matrix element $Q_{i,j}$ to the annealing computer 10 as a parameter of the Ising model.

$$E = \Sigma Q_{i,j} n_i n_j + \Sigma Q_{ii} n_i \quad \cdots \quad (6)$$

In step S16, the optimum solution computing unit 22 of the annealing computer 10, that receives the parameter of the Ising model, obtains $n_{i,j}$ with which the Hamiltonian of the formula (1) becomes a minimum, as the optimum solution. $n_{i,j}$ with which the Hamiltonian of the formula (1) becomes the minimum, represents the optimum solution of the mixing ratio of the mixed solvent having the objective physical property asymptotically approaching the target value.

As described above, $n_{i,j}$ is a variable in which a number "0" or "1" is stored when the mixing ratio of the solvent i is expressed in binary. $n_{i,j}$ is "0" when the solvent i is included in the ratio $c_j$, and is "1" when the solvent i is not included in the ratio $c_j$. In a case where the solvent i amounting to 33% is included, for example, $\{n_{i,j}\} = (1, 0, 1, 1, 1, 1, 0)$ according to the third expression method of the mixing ratio described above.

In step S18, the coordination unit 36 of the material composition searching device 12 receives $n_{i,j}$ with which the Hamiltonian of the formula (1) described above, obtained by the annealing computer 10, becomes the minimum, as an optimum solution.

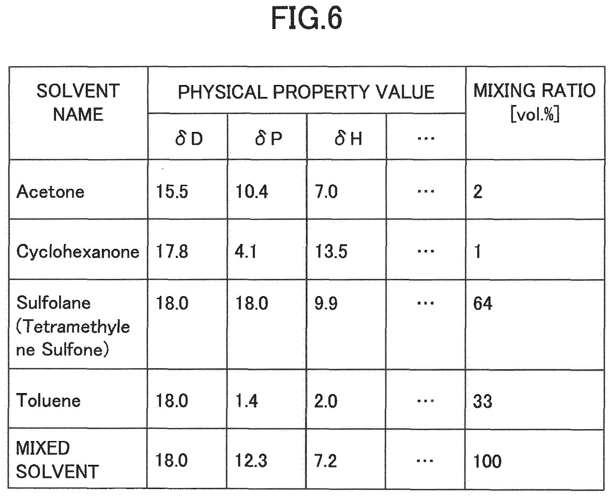

The output control unit 38 converts $n_{i,j}$, that is received by the coordination unit 36 as the optimum solution, into information which is easy for the user to understand and outputs the information. The output control unit 38 can output the information of the mixed solvent, that is the optimum solution illustrated in FIG. 6, for example, from $n_{i,j}$ that is received as the optimum solution.

FIG. 6 is a diagram illustrating a structure of examples of combinations of solvents that become an optimum solution, and mixing ratios. FIG. 6 illustrates an example of information including the solvents included in the mixed solvent of the optimum solution, the mixing ratios of the solvents, and the physical property values of the mixed solvent. The physical property value of the mixed solvent can be computed from the formula (3) described above. In addition, in the example of FIG. 6, for example, by mixing four kinds of solvents, namely, acetone, cyclohexanone, sulfolane (tetramethylene sulfone), and toluene, at a ratio 2:1:64:33, the objective physical properties $\delta D$, $\delta P$, and $\delta H$ of the mixed solvent become close to the target values $(\delta D_0, \delta P_0, \delta H_0) = (18.0, 12.3, 7.2)$ of the objective physical properties $\delta D$, $\delta P$, and $\delta H$.

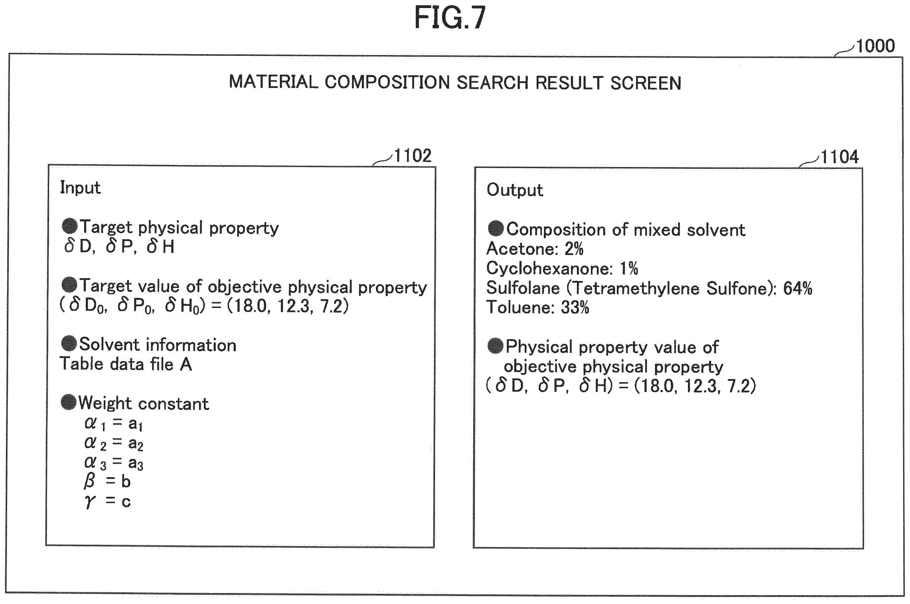

For example, the output control unit 38 may output the information on the mixed solvent that becomes the optimum solution to a material composition search result screen 1000 illustrated in FIG. 7. FIG. 7 is a diagram illustrating an image of an example of the material composition search result screen. The material composition search result screen 1000 displays a display field 1102 for the information input by the user in order to cause the annealing machine to solve the combinatorial optimization problem that is reduced to the Ising model, and a display field 1104 for outputting the information on the mixed solvent that becomes the optimum solution.

In the display field 1102, an objective physical property, a target value of the objective physical property, solvent information, and a weight constant are displayed, as examples of the information input by the user. In addition, in the display field 1104, a composition of the mixed solvent (a kind of solvent included in the mixed solvent, and a mixing ratio of the solvent) and a physical property value of an objective physical property are displayed, as examples of the information on the mixed solvent that becomes the optimum solution.

By checking the material composition search result screen 1000 of FIG. 7, the user can easily evaluate the mixed solvent that is obtained as the optimum solution. In addition, as a result of checking the material composition search result screen 1000 of FIG. 7, if the user desires to search for another mixed solvent, the user may readjust the weight constants included in the formula (1) described above, for example, and then perform the processes of the flow chart illustrated in FIG. 5. The readjustment of the weight constants may be performed manually by the user, or may be performed using a program or the like that automatically optimizes the weight constants.

If the user desires to decrease the number of kinds of solvents included in the mixed solvent, for example, the user can increase the weight constant $\gamma$ of the formula (1) described above, so that a minimum value of the formula (1) is obtained when the number of solvents included in the mixed solvent is small, even when the objective physical property is slightly deviated from the target value, and the solvent composition desired by the user is more likely computed as the optimum solution.

The material composition search result screen 1000 of FIG. 7 is an example, and may be provided with a field for readjusting the weight constants included in the formula (1) described above, a search-again button for performing the process of searching-again the optimum solution using the formula (1) described above after the weight constants are readjusted, or the like. The material composition search result screen 1000 of FIG. 7 may be displayed together with the diagram of the structure of FIG. 6.

The information on the mixed solvent searched as the optimum solution can be used to control a mixed solvent generating apparatus or the like, for example, that generates the mixed solvent, by specifying the solvents to be mixed and the mixing ratios thereof. In addition, the physical properties of the mixed solvent generated by the mixed solvent generating apparatus can be evaluated by an evaluation apparatus. Hence, the information on the mixed solvent searched as the optimum solution can be compared with the physical properties of the mixed solvent generated by the mixed solvent generating apparatus by specifying the information on the mixed solvent, and the result of the comparison can be fed back so as to improve the accuracy of the comparison.

As described above, in the information processing system 1 according to this embodiment, it is possible to artificially vary the optimum solution of the combinatorial optimization problem of the composition of the mixed solvent asymptotically approaching the target value of the objective physical property, according to the number of solvents included in the mixed solvent.

Although the embodiments are described heretofore, various modifications to the form and details may be made without departing from the subject matter and scope of the appended claims.

Although the present invention is described with reference to the embodiments, the present invention is not limited to the embodiments described above, and various modifications can be made without departing from the scope of the subject matter recited in the claims. The present international application is based upon and claims priority to Japanese Patent Application No. 2021-097381, filed on June 10, 2021, the entire contents of which are incorporated herein by reference.

DESCRIPTION OF REFERENCE NUMERALS

1 Information processing system
10 Annealing computer
12 Material composition searching device
18 Communication network
20 Call reception unit
22 Optimum solution computing unit
30 Input reception unit
32 Formulation unit
34 Conversion unit
36 Coordination unit
38 Output control unit
40 Solvent information storage unit
42 Mathematical expression storage unit
502 Display device

**Claims**

1. An information processing system having an annealing computing device configured to use an Ising model, and a material composition searching device configured to convert a combinatorial optimization problem of a material composition asymptotically approaching a target physical property value into an Ising model and cause the computing device to solve the combinatorial optimization problem, **characterized in that** there are provided:

   an input reception unit configured to receive an input of a target value of at least one physical property;
   a conversion unit configured to convert a mathematical expression that formulates a combinatorial optimization problem of a material composition that asymptotically approaches the target value from a mixed material of materials having known physical property values, into the Ising model in a data format usable by the computing device;
   an optimum solution computing unit configured to compute an optimum solution of the material composition asymptotically approaching the target value, using the Ising model; and
   an output control unit configured to output the computed optical solution of the material composition asymptotically approaching the target value,
   wherein the mathematical expression formulates the material composition that asymptotically approaches the target value, so that the optimum solution is less likely computed as a number of materials included in the material composition increases, and is more likely computed as the number of materials included in the material composition decreases.

2. The information processing system as claimed in claim 1, **characterized in that** the mathematical expression formulates the combinatorial optimization problem of the material composition asymptotically approaching the target value using an energy function including

   a cost function that outputs a value so that the value is more likely computed as the optimum solution as the physical property value of the mixed material becomes closer to the target value, and the value is less likely computed as the optimum solution as the physical property value of the mixed material becomes farther from the target value,
a first constraint condition that outputs a value so that the value is not computed as the optimum solution in a case where a total of mixing ratios of materials included in the mixed material does not become 100%, and
a second constraint condition that outputs a value so that the value is more likely computed as the optimum solution as the number of materials included in the material composition decreases, and the value is less likely computed as the optimum solution as the number of materials included in the material composition increases.

3. The information processing system as claimed in claim 1 or 2, **characterized in that** the mathematical expression is the following formula (1),
[Math. 1]

$$E = \sum_{k=1}^{L} \alpha_k \cdot \left\{ \left( \sum_{i=1}^{N} D_{k,i} r_i \right) - D_{k,0} \right\}^2 + \beta \left\{ \left( \sum_{i=1}^{N} r_i \right) - 1 \right\}^2 + \gamma \left\{ N - \sum_{i=1}^{N} \left( \prod_{j}^{m} n_{i,j} \right) \right\} \cdots (1)$$

where in the formula (1),

L denotes a number of objective physical properties to be optimized,
N denotes a number of solvents with known physical property values,
$D_{k,i}$ denotes a physical property value of a k-th objective physical property of a solvent i to be optimized,
$r_i$ denotes the following formula (2) in which the mixing ratio (component ratio) of the solvent i is expressed in binary,
[Math. 2]

$$r_i = -\sum_{j=1}^{m} c_j (n_{i,j} - 1) \cdots (2)$$

$D_{k,0}$ denotes a target value of the k-th objective physical property of the mixed solvent to be optimized,
$n_{i,j}$ denotes a number "0" or "1" for a case where the mixing ratio of the solvent i is expressed in binary,
$c_j$ denotes a coefficient for the case where the mixing ratio of the solvent i is expressed in binary, and
$\alpha_k$, $\beta$, and $\gamma$ denote weight constants.

4. The information processing system as claimed in claim 3, **characterized in that** the optimum solution computing unit computes a combination of $n_{i,j}$ with which the energy function of the formula (1) becomes a minimum value, as the optimum solution of the material composition asymptotically approaching the target value, using the Ising model obtained by converting the formula (1) into the data format usable by the computing device.

5. The information processing system as claimed in any one of claims 1 to 4, **characterized in that**

the input reception unit receives, from a user, a selection of a target value of at least one physical property, and a selection of a material to be used as the material composition of the mixed material from a plurality of materials having the at least one physical property with a known physical property value, and
the output control unit converts the optimum solution of the material composition computed by the optimum solution computing unit into at least one of a material included in the material composition, a mixture ratio of the material, and a physical property value of the mixed material.

6. A material composition searching method executed by an information processing system having an annealing computing device configured to use an Ising model, and a material composition searching device configured to convert a combinatorial optimization problem of a material composition asymptotically approaching a target physical property value into an Ising model and cause the computing device to solve the combinatorial optimization problem, **characterized in that** there are provided:

step of receiving an input of a target value of at least one physical property;
step of converting a mathematical expression that formulates a combinatorial optimization problem of a material composition that asymptotically approaches the target value from a mixed material of materials having known physical property values, into the Ising model in a data format usable by the computing device;

step of computing an optimum solution of the material composition asymptotically approaching the target value, using the Ising model; and

step of outputting the computed optical solution of the material composition asymptotically approaching the target value,

wherein the mathematical expression formulates the material composition that asymptotically approaches the target value, so that the optimum solution is less likely computed as a number of materials included in the material composition increases, and is more likely computed as the number of materials included in the material composition decreases.

7. A material composition searching device that is coupled to an annealing computing device configured to use an Ising model via a communication network, and converts a combinatorial optimization problem of a material composition asymptotically approaching a target physical property value into an Ising model and causes the computing device to solve the combinatorial optimization problem, **characterized in that** there are provided:

an input reception unit configured to receive an input of a target value of at least one physical property;

a conversion unit configured to convert a mathematical expression that formulates a combinatorial optimization problem of a material composition that asymptotically approaches the target value from a mixed material of materials having known physical property values, into the Ising model in a data format usable by the computing device;

a coordination unit configured to transmit the converted Ising model to the computing device, and receive, from the computing device, an optimum solution of a material composition asymptotically approaching the target value computed by the computing device; and

an output control unit configured to output the received optical solution of the material composition asymptotically approaching the target value,

wherein the mathematical expression formulates the material composition that asymptotically approaches the target value, so that the optimum solution is less likely computed as a number of materials included in the material composition increases, and is more likely computed as the number of materials included in the material composition decreases.

8. A program for causing a material composition searching device that is coupled to an annealing computing device configured to use an Ising model via a communication network, and converts a combinatorial optimization problem of a material composition asymptotically approaching a target physical property value into an Ising model and causes the computing device to solve the combinatorial optimization problem, to function as:

an input reception unit configured to receive an input of a target value of at least one physical property;

a conversion unit configured to convert a mathematical expression that formulates a combinatorial optimization problem of a material composition that asymptotically approaches the target value from a mixed material of materials having known physical property values, into the Ising model in a data format usable by the computing device;

a coordination unit configured to transmit the converted Ising model to the computing device, and receive, from the computing device, an optimum solution of a material composition asymptotically approaching the target value computed by the computing device; and

an output control unit configured to output the received optical solution of the material composition asymptotically approaching the target value,

wherein the mathematical expression formulates the material composition that asymptotically approaches the target value, so that the optimum solution is less likely computed as a number of materials included in the material composition increases, and is more likely computed as the number of materials included in the material composition decreases.

# FIG.1

1

10

ANNEALING COMPUTER

18

12

MATERIAL
COMPOSITION
SEARCHING DEVICE

# FIG.2

EP 4 354 362 A1

# FIG.3

| SOLVENT NAME | PHYSICAL PROPERTY VALUE | | | |
|---|---|---|---|---|
| | $\delta D$ | $\delta P$ | $\delta H$ | ... |
| Acetone | 15.5 | 10.4 | 7.0 | ... |
| Cyclohexanone | 17.8 | 4.1 | 13.5 | ... |
| Sulfolane (Tetramethylene Sulfone) | 18.0 | 18.0 | 9.9 | ... |
| Toluene | 18.0 | 1.4 | 2.0 | ... |
| ... | ... | ... | ... | ... |

# FIG.4

1

ANNEALING COMPUTER ~10

OPTIMUM SOLUTION COMPUTING UNIT ~22

CALL RECEPTION UNIT ~20

~18

MATERIAL COMPOSITION SEARCHING DEVICE ~12

INPUT RECEPTION UNIT ~30

FORMULATION UNIT ~32

CONVERSION UNIT ~34

COORDINATION UNIT ~36

OUTPUT CONTROL UNIT ~38

SOLVENT INFORMATION STORAGE UNIT ~40

MATHEMATICAL EXPRESSION STORAGE UNIT ~42

# FIG.5

```
                    ( START )
                        │
                        ▼                          S10
┌─────────────────────────────────────────────────┐
│          RECEIVE INPUTS OF OBJECTIVE PHYSICAL     │
│       PROPERTY OF MIXED SOLVENT TO BE OPTIMIZED,  │
│       TARGET VALUE OF OBJECTIVE PHYSICAL PROPERTY,│
│                 AND WEIGHT CONSTANTS              │
│        (FOR EXAMPLE, VISCOSITY, HSP, COST, ETC.)  │
└─────────────────────────────────────────────────┘
                        │
                        ▼                          S12
┌─────────────────────────────────────────────────┐
│          RECEIVE INPUT OF SOLVENT INFORMATION     │
│                 OF SOLVENTS TO BE MIXED           │
└─────────────────────────────────────────────────┘
                        │
                        ▼                          S14
┌─────────────────────────────────────────────────┐
│                CONVERT INTO ISING MODEL           │
│   (COMPUTE MATRIX ELEMENTS OF FORMULA OF ISING MODEL) │
│    HAVING DATA FORMAT USABLE IN ANNEALING COMPUTER│
│          (SUITABLE FOR ANNEALING COMPUTER),       │
│            USING OBJECTIVE PHYSICAL PROPERTY,      │
│       TARGET VALUE OF OBJECTIVE PHYSICAL PROPERTY, │
│                  AND FORMULA (1)                  │
└─────────────────────────────────────────────────┘
                        │
                        ▼                          S16
┌─────────────────────────────────────────────────┐
│  COMPUTE OPTIMUM SOLUTION OF SOLVENT COMPOSITION, │
│          USING ISING MODEL HAVING DATA FORMAT     │
│              USABLE IN ANNEALING COMPUTER         │
└─────────────────────────────────────────────────┘
                        │
                        ▼                          S18
┌─────────────────────────────────────────────────┐
│      OUTPUT MIXED SOLVENT OF OPTIMUM SOLUTION,    │
│            AND PHYSICAL PROPERTY VALUE OF         │
│    OBJECTIVE PHYSICAL PROPERTY OF MIXED SOLVENT   │
└─────────────────────────────────────────────────┘
                        │
                        ▼
                    ( END )
```

# FIG.6

| SOLVENT NAME | PHYSICAL PROPERTY VALUE | | | | MIXING RATIO [vol.%] |
|---|---|---|---|---|---|
| | $\delta$ D | $\delta$ P | $\delta$ H | $\cdots$ | |
| Acetone | 15.5 | 10.4 | 7.0 | $\cdots$ | 2 |
| Cyclohexanone | 17.8 | 4.1 | 13.5 | $\cdots$ | 1 |
| Sulfolane (Tetramethylene Sulfone) | 18.0 | 18.0 | 9.9 | $\cdots$ | 64 |
| Toluene | 18.0 | 1.4 | 2.0 | $\cdots$ | 33 |
| MIXED SOLVENT | 18.0 | 12.3 | 7.2 | $\cdots$ | 100 |

# FIG.7

**MATERIAL COMPOSITION SEARCH RESULT SCREEN** — 1000

### 1102

Input

● Target physical property
$\delta D$, $\delta P$, $\delta H$

● Target value of objective physical property
$(\delta D_0$, $\delta P_0$, $\delta H_0) = (18.0, 12.3, 7.2)$

● Solvent information
Table data file A

● Weight constant
$\alpha_1 = a_1$
$\alpha_2 = a_2$
$\alpha_3 = a_3$
$\beta = b$
$\gamma = c$

### 1104

Output

● Composition of mixed solvent
Acetone: 2%
Cyclohexanone: 1%
Sulfolane (Tetramethylene Sulfone): 64%
Toluene: 33%

● Physical property value of
objective physical property
$(\delta D$, $\delta P$, $\delta H) = (18.0, 12.3, 7.2)$

## INTERNATIONAL SEARCH REPORT

| International application No. |
|---|
| **PCT/JP2022/022916** |

| **A.** | **CLASSIFICATION OF SUBJECT MATTER** |
|---|---|

***G06N 99/00***(2019.01)i
FI:   G06N99/00 180

According to International Patent Classification (IPC) or to both national classification and IPC

| **B.** | **FIELDS SEARCHED** |
|---|---|

Minimum documentation searched (classification system followed by classification symbols)

G06N99/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| A | JP 2020-30683 A (YOKOHAMA RUBBER CO., LTD.) 27 February 2020 (2020-02-27) entire text, all drawings | 1-8 |
| A | 田村 亮, マテリアルズ・インフォマティクスにおける量子アニーリングの活用, 電子情報通信学会2019年基礎・境界ソサイエティ／NOLTAソサイエティ大会講演論文集, 27 August 2019, SS-47, non-official translation (TAMURA, Ryo. Application of quantum annealing in materials informatics. Proceedings of the 2019 Fundamental and Boundary Society/NOLTA Society Conference of The Institute of Electronics, Information and Communication Engineers.) sections 2, 3 | 1-8 |
| P, A | JP 2021-127418 A (FUJITSU LTD.) 02 September 2021 (2021-09-02) paragraphs [0020], [0050]-[0052], [0076]-[0079], [0145]-[0147], fig. 7, 9 | 1-8 |

☑ Further documents are listed in the continuation of Box C.     ☑ See patent family annex.

```
*    Special categories of cited documents:
"A"  document defining the general state of the art which is not considered
     to be of particular relevance
"E"  earlier application or patent but published on or after the international
     filing date
"L"  document which may throw doubts on priority claim(s) or which is
     cited to establish the publication date of another citation or other
     special reason (as specified)
"O"  document referring to an oral disclosure, use, exhibition or other
     means
"P"  document published prior to the international filing date but later than
     the priority date claimed
```

```
"T"  later document published after the international filing date or priority
     date and not in conflict with the application but cited to understand the
     principle or theory underlying the invention
"X"  document of particular relevance; the claimed invention cannot be
     considered novel or cannot be considered to involve an inventive step
     when the document is taken alone
"Y"  document of particular relevance; the claimed invention cannot be
     considered to involve an inventive step when the document is
     combined with one or more other such documents, such combination
     being obvious to a person skilled in the art
"&"  document member of the same patent family
```

| Date of the actual completion of the international search | Date of mailing of the international search report |
|---|---|
| **26 August 2022** | **06 September 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| | International application No. |
|---|---|
| | **PCT/JP2022/022916** |

**C.     DOCUMENTS CONSIDERED TO BE RELEVANT**

| Category* | Citation of document, with indication, where appropriate, of the relevant passages | Relevant to claim No. |
|---|---|---|
| P, A | ニュースリリース「量子コンピューティング技術の活用により半導体材料の最適配合探索の大幅な高速化を実現」, [online], 昭和電工株式会社, 10 February 2022, [retrieved on 23 August 2022], Internet:<URL:https://www.sdk.co.jp/news/2022/41712.html>, non-official translation (News release: Utilization of quantum computing technology realizes a drastic speed-up in the search for optimal compounding of semiconductor materials. SHOWA DENKO KK.)<br>       entire text, all drawings | 1-8 |

Form PCT/ISA/210 (second sheet) (January 2015)

## INTERNATIONAL SEARCH REPORT
### Information on patent family members

| | International application No. |
|---|---|
| | **PCT/JP2022/022916** |

| Patent document cited in search report | | | Publication date (day/month/year) | Patent family member(s) | Publication date (day/month/year) |
|---|---|---|---|---|---|
| JP | 2020-30683 | A | 27 February 2020 | (Family: none) | |
| JP | 2021-127418 | A | 02 September 2021 | US 2021/0256185 A1 paragraphs [0040]-[0042], [0105]-[0115], [0165]-[0177], [0295]-[0304], fig. 7, 9<br>EP 3866170 A1<br>CN 113268126 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021097381 A

**Non-patent literature cited in the description**

- Optimization of Thermophysical Properties for Mixed Refrigerants with Digital Annealer. The Proceedings of the Thermal Engineering Conference 2019. The Japan Society of Mechanical Engineers, 12 October 2019